(19) Europäisches Patentamt
European Patent Office
Office européen des brevets

(11) EP 2 966 422 A1

(12) **EUROPEAN PATENT APPLICATION**
published in accordance with Art. 153(4) EPC

(43) Date of publication:
**13.01.2016 Bulletin 2016/02**

(21) Application number: **14759872.6**

(22) Date of filing: **04.03.2014**

(51) Int Cl.:
***G01G 3/13*** (2006.01) ***A61B 5/22*** (2006.01)
***A43B 17/00*** (2006.01) ***G06F 19/00*** (2011.01)

(86) International application number:
**PCT/RU2014/000138**

(87) International publication number:
**WO 2014/137245 (12.09.2014 Gazette 2014/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.03.2013 RU 2013110574**

(71) Applicant: **Healbe Corporation**
**Redwood City, CA 94063 (US)**

(72) Inventors:
- **RUBIN, Mikhail Semenovich**
**St.Petersburg 191119 (RU)**
- **MISJUCHENKO, Igor Leonidovich**
**St.Petersburg 197371 (RU)**
- **GERASIMOV, Oleg Mikhailovich**
**Sertolovo**
**Leningradskaya obl. 188650 (RU)**

(74) Representative: **Reichert & Lindner Partnerschaft Patentanwälte**
**Bismarckplatz 8**
**93047 Regensburg (DE)**

(54) **METHOD FOR DETERMINING AN INDIVIDUAL'S WEIGHT AND INSOLE FOR THE IMPLEMENTATION THEREOF**

(57) A method for measuring parameters, such as human weight, together with additionally carried weight, in motion. The method provides registration of signals generated by load sensors disposed in shoe insoles; whereat each insole has at least two load sensors, with one mounted near the heel region and the other near the toe region of foot. The specific type of motor activity is determined based on temporal correlation of the load sensor signals from both insoles and values thereof. The person's weight, including additionally carried weight, is determined by summing up load sensor signals, for a specific type of motor activity. The invention provides for the measurement of person's weight, including additionally carried weight, in real time for different types of motor activity, such as running, walking at different pace, standing.

3
1
4
5
4
2

1
3
4
5
4
2

Fig. 1

EP 2 966 422 A1

## Description

### FIELD OF INVENTION

The invention relates to the field of measurements of bodily parameters in motion for diagnostic purposes, in particular to measuring human weight, including additionally carried weight, using load sensors located in shoe insoles.

### BACKGROUND OF THE INVENTION

Various methods are known for determining the weight of a human in motion, which are implemented using pressure sensors disposed in shoes.

Application JP 2002090216 (publication date Sep. 11, 2000; G01G19/52, A61B5/11) describes a device that allows for determining person's weight applied to his/her bad leg. The device is designed as a special shoe with a bottom plate and a top plate made as a shoe sole capable of moving with respect to the lower plate. An air bag is placed between the plates, wherein air pressure varies depending on the weight applied to the foot. A measuring device in the upper part of the shoe measures the pressure in the air bag with the value thereof used to estimate the weight.

International patent application WO 200136051 (publication date May, 05, 2001, A63B05/103) describes a device, which implements a method for measuring human weight applied, at least, to one limb. The device is designed to train patients in distributing their weight between a healthy leg and a bad one, and can be used to rehabilitate patients with neurological disorders or orthopedic trauma. The device includes flexible insoles with pressure sensors located near the heel and toe of the foot. Signals from the sensors are processed by a CPU, which generates signals indicating proper or improper distribution of patient's weight on non-healthy leg.

US Patent 6273863 (publication date Aug. 14, 2001; A61B05/103, A61B05/117) describes a monitoring system for rehabilitating orthopedic patients with fractures of the lower extremities. The system includes a flexible insole worn inside the shoe. The insole includes pressure and/or force sensor connected to a CPU to measure the weight applied to injured leg. The system incorporates the principle of biofeedback to encourage the patient to load the limb with the optimal target weight recommended for the period of rehabilitation.

The closest to the claimed invention is the method and device for measuring weight of a human, as described in international patent application WO2009059134 (publication date May 05, 2009; A61B05/103, G01L01/00). That concept makes it possible to monitor the weight applied by a patient to a non-healthy leg in order to prevent the exceedance of a preset threshold of limb load for a given patient. For this purpose, electrical or mechanical pressure sensors are mounted on the support surface. The sensors are located near the heel and toe of the foot, as well as across the entire surface of the foot sole. The support surface can be placed in shoes. Sensor signals are processed by a microcontroller which determines whether the weight applied to a non-healthy leg exceeds a preset threshold.

Known solutions for measuring the weight of a human are primarily intended for medical purposes, in particular, for measuring the weight applied by a patient to his/her non-healthy leg. This predetermines the complex design of the devices used therefor and restricts their application primarily to medical purposes.

The technical problem solved by the present invention is to develop a method for determining weight of a human, especially the weight of a healthy human. A real-time determination of not only a human's own weight, but also of the weight additionally carried by the human, is enabled for different types of motor activities. The inventive method makes it possible to assess various physiological parameters of a human condition, including those related to the motor activity.

### SUMMARY OF THE INVENTION

One of the objects of this invention is a method for determining the weight of a human using signals generated by load sensors disposed in shoe insoles, with each insole having two load sensors, a first load sensor disposed in the heel region, and the second load sensor disposed in the toe region of the foot. The specific type of motor activity is determined based on the signals and their values from the load sensors from both insoles. The weight of a human, including additionally carried weight, is determined by summing up the load sensor signals and based on the type of the human's motor activity.

During a motor activity, a human steps on the legs alternatingly, first on one, then the other leg. The load sensors located in the heel region and in the toe region make it possible to determine the duration of the contact of the foot with a base (support phase) and the duration of leg transfer (transfer phase) within one walking or running cycle. Since different types of motor activity are characterized by different correspondence in time between the support time and the transfer time, the correlation between the signals of the load sensors from different insoles lets one determine a pattern or a type of a motor activity.

Specifically, the present method provides for determining the weight of a human, including additionally carried weight, directly during the motor activity (walking, running, etc.) and in accordance with the type of the motor activity. That approach provides for the assessment of various physiological parameters of a human condition, including those related to a motor activity.

In particular implementations of this method, various types of a motor activity can be determined as follows.

Such type of a motor activity as "walking" is identified if the signals from the load sensors in both insoles exhibit periodic variations of the values of the signals and the signals from the load sensors of different insoles partially overlap in time.

The type of the motor activity is determined as "running" if the signals from the load sensors in both insoles exhibit periodic variations of the values of the signals and the signals from the load sensors of different insoles do not overlap in time.

The type of the motor activity is determined as "standing" if the signals from the load sensors in both insoles exceed a predetermined value and the signals from different insoles overlap in time.

The inventors have obtained a series of experimental dependencies that provide for the determination of the weight of a human, including additionally carried weight, in consideration with the specific type of a motor activity.

For example, weight *P*, including additionally carried weight, of a person/human, who is walking at a slow pace of up to 60 steps per minute can be expressed as follows:

$$P = K_W \cdot F \,,$$

where: $K_W$ is a calibration factor determined at walking for a person with a known weight;

*F* is the mean value of a foot pressure force over one walking cycle, wherein:

$$F = (F_{1\max} + F_{2\max})/2 \,,$$

where: $F_{1max}$ is the maximum value of summarized foot pressure forces registered by all load sensors in one insole;

$F_{2max}$ is the maximum value of summarized foot pressure forces registered by all load sensors in the other insole,

wherein one cycle consists of two consecutive steps made by one foot and then the other.

Weight *P*, including additionally carried weight, of a person, who is walking at a pace of 60 or more steps per minute can be expressed as follows:

$$P = K_W \cdot F \cdot (1010 - 1.2 \cdot V - 0.026 \cdot V^2) \cdot 0.001 \,,$$

where: $K_W$ is a calibration factor determined at walking with pace of up to 60 steps per minute for a given person with a known weight;

*F* is the mean value of a foot pressure force over one walking cycle, wherein:

$$F = (F_{1\max} + F_{2\max})/2 \,,$$

where: $F_{1max}$ is the maximum value of summarized foot pressure forces registered by all load sensors in one insole;

$F_{2max}$ is the maximum value of summarized foot pressure forces registered by all the load sensors in the other insole;

*V* is the number of steps per minute,

wherein one cycle consists of two consecutive steps made by one foot and then the other.

The weight, including additionally carried weight, of a person during running can be expressed by formula:

$$P = K_R \cdot F \cdot (1090 - 4.4 \cdot V - 0.045 \cdot V^2) \cdot 0.001,$$

where: $K_R$ is a calibration factor determined at running for a given person with a known weight;

$F$ is the mean value of a foot pressure force over one running cycle, wherein:

$$F = (F_{1\max} + F_{2\max})/2,$$

where: $F_{1max}$ is the maximum value of summarized foot pressure forces registered by all load sensors in one insole;

$F_{2max}$ is the maximum value of summarized foot pressure forces registered by all load sensors in the other insole;

$V$ is the number of steps per minute,

wherein one running cycle is assumed to consist of two consecutive steps made by one foot and the other.

The weight, including additionally carried weight, of a person during standing can be expressed as:

$$P = K_S \cdot F,$$

where: $K_S$ is a calibration factor determined during standing for a given person with a known weight;

$F$ is the mean value of a foot pressure force over the period of standing, wherein:

$$F = (F_{1\max} + F_{2\max})/2,$$

where: $F_{1max}$ is the maximum value of summarized foot pressure forces registered by all load sensors in one insole;

$F_{2max}$ is the maximum value of summarized foot pressure forces registered by all load sensors in the other insole.

Specifically, the method also contemplates providing additional load sensors in each insole, the additional sensors being disposed along a trajectory of a support reaction force at walking between the first load sensor and the second load sensor, detecting signals from the additional load sensors and using such signals in addition to using the signals from the first and the second load sensors.

Another object of the invention is to provide an insole for determining the weight of the human according to the inventive method, the insole comprising a first load sensor disposed in the insole in a heel region, and a second load sensor disposed in the insole in a toe region, the first and the second load sensors being capable of generating signals detecting a pressure force applied by the person's foot.

Additionally, the insole further comprises an analog-to-digital converter and a transceiver to convert the signals from the load sensors to a digital form and transmit them to an external processing unit.

## BRIEF DESCRIPTION OF THE DRAWINGS

The invention is illustrated by the following graphic materials.

Fig. 1 is a schematic illustration of the design of the insole with load sensors mounted therein: shown here are both, left and right insoles and location of load sensors relative to the trajectory of support reaction force at walking;

Fig. 2 shows a wiring diagram of an exemplary device for measuring the force applied by foot upon load sensors mounted in insoles, as shown in Fig. 1: each load sensor is realized as a strain transducer with four bridge-connected strain gages;

Fig. 3 shows exemplary timing diagrams which illustrate the temporal relationship of load sensor signals from both insoles as registered by the circuit from Fig. 2 in case of standing activity.

Fig. 4 shows exemplary timing diagrams which illustrate the temporal relationship of load sensor signals from both insoles as registered by the circuit from Fig. 2 in case of walking activity.

Fig. 5 shows exemplary timing diagrams which illustrate the temporal relationship of load sensor signals from both insoles as registered by the circuit from Fig. 2 in case of running activity.

Fig. 6 shows a circuit diagram of another exemplary device for measuring the force applied by foot upon load sensors placed in insole, as shown in Fig. 1: each load sensor is realized as a piezoelectric transducer.

Fig. 7 shows exemplary timing diagrams which illustrate the temporal relationship of load sensor signals from both insoles as registered by the circuit from Fig. 6 in case of walking activity.

Fig. 8 shows exemplary timing diagrams which illustrate the temporal relationship of load sensor signals from both insoles as registered by the circuit from Fig. 6 in case of running activity.

## DETAILED DESCRIPTION OF THE PREFERRED EMBODIMENTS

The present invention is illustrated by the following embodiments of insoles with load sensors realized as strain gages and piezoelectric transducers.

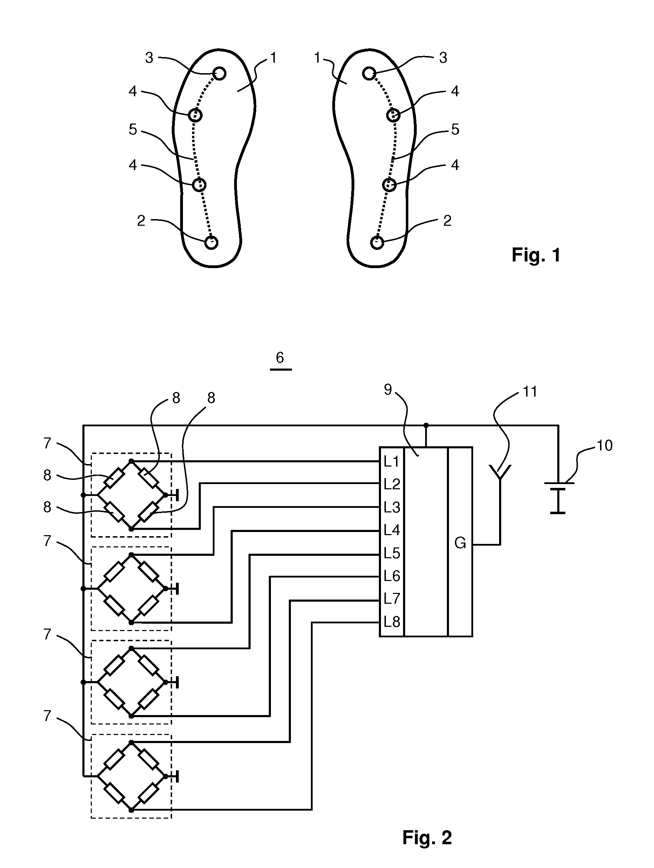

In the first embodiment, each insole 1 (see. Fig. 1) includes a first load sensor 2 located near the heel of the foot, a second load sensor 3 located near the toe of the foot, and two additional load sensors 4 located actually along the trajectory 5 of support reaction forces at walking. Fig. 2 shows an exemplary schematic diagram of device 6 for registering the force applied upon load sensor by foot. In this embodiment, load sensors 2-4 are represented by strain transducers (strain sensors) 7 each consisting of four bridge-connected strain gages 8. One bridge diagonal is connected to power supply 10, while the other is connected to input/output ports of microcontroller 9 in a way that all four strain transducers 7 (first load sensor 2, second load sensor 3, and two additional load sensors 4) are eventually connected to eight input/output ports L1-L8 of microcontroller 9 which registers analog signals from strain transducers 7 and converts them to digital form. Antenna output G of microcontroller 9 equipped with a built-in transceiver is connected to antenna 11. Device 6 is energized by power supply 10. Microcontroller 9 is switched into operating mode by commands from an external computer (not shown in the drawings). The external computer is mounted in a man-portable device and is also capable of determining exercise stress.

The method of human exercise stress monitoring according to the present invention is executed as follows.

The type of motor activity is determined using signals from strain gages 7 (load sensors 2-4) of both insoles 1, which are registered by signal processing unit 6, and time relationship of said signals from right and left insoles 1.

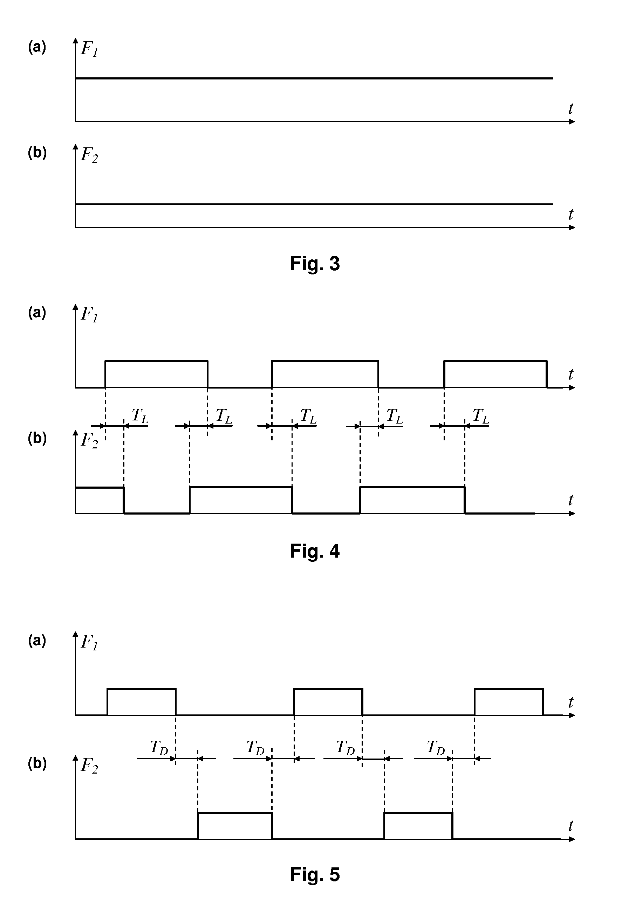

For example, such activity as standing is distinguished by signals shown in Fig. 3, where graph (a) corresponds to signal $F_1$ from strain gage 7 of insole 1, while graph (b) corresponds to signal $F_2$ from strain gage 7 of the other insole 1. At this type of motor activity, signals $F_1$ and $F_2$ from strain gages 7 of both insoles 1 are essentially equal in value and feature almost complete overlap in time. Fig. 3 gives an example in which signal $F_1$ from strain gage 7 of insole 1 (graph (a)) is greater in value than signal $F_2$ from strain gage 7 of the other insole 1 (graph (b)). It means that the person is standing leaning on one leg more than the other.

Such type of motor activity as walking is characterized by signals shown in Fig. 4, where graph (a) corresponds to signal $F_1$ from strain gage 7 of insole 1, while graph (b) corresponds to signal $F_2$ from strain gage 7 of the other insole 1. This type of motor activity is characterized by alternating variation of signal values $F_1$ and $F_2$ from strain gages 7 of both insoles 1, and by the availability of partial overlap in time (overlap time interval $T_L$).

Such type of motor activity as running is characterized by signals shown in Fig. 5, where graph (a) corresponds to signal $F_1$ from strain gage 7 of insole 1, while graph (b) corresponds to signal $F_2$ from strain gage 7 of the other insole 1. This type of motor activity is characterized by alternating variation of signal values $F_1$ and $F_2$ from strain gages 7 of both insoles 1, and by the absence of time overlap of said signals. Conversely, a time gap between these signals designated as time interval $T_D$ is observed.

The availability of at least two load sensors in each insole, with the first sensor 2 placed near the heel and the second sensor 3 near the toe, makes it possible to define not only the above-mentioned types of motor activity (walking, running, standing), but also other types like sitting, cycling, skiing. However, this method covers only those types of motor activity in which person's weight, including additionally carried weight, is transmitted to the feet.

The table below matches load sensor signal values and their temporal relationship to a certain type of motor activity (with at least two sensors placed in one insole).

| Load sensor signal values from both insoles and their temporal relationship | Type of human motor activity |
|---|---|
| Not available | Sitting, lying, with shoes removed |
| Essentially invariable values and almost complete time overlap | Standing |
| Partial overlap at cyclic repetition | Walking |
| No overlap at cyclic repetition | Running |
| Cyclic and essentially simultaneous change of sensor signal values from both insoles | Jumping |

Similar signals from load sensors 2, 3, and 4 used to determine (identify) the type of person's motor activity can be also used to measure person's weight, including additionally carried weight. The term "additionally carried weight" means extra weight borne by the person, for example carried items or special training weights.

The mathematical relationships experimentally obtained by the inventors with due regard to the type of motor activity can be used for measuring person's weight, including additionally carried one.

Thus, at standing activity the weight measurement is basically reduced to the summation of signal values from all load sensors 2-4 of both insoles.

The weight, including additionally carried weight, of a standing person can be determined by formula:

$$P = K_S \cdot F\,,$$

where: $K_S$ is a calibration factor to be determined for a given person with a known weight at standing;

$F$ is the mean value of pressure force of feet at standing. It can be determined for a certain period of time, e.g., from 5 to 10 seconds, wherein:

$$F = (F_{1\max} + F_{2\max})/2\,,$$

where: $F_{1max}$ is the maximum value of summarized foot pressure forces registered by all load sensors of one insole;

$F_{2max}$ is the maximum value of summarized foot pressure forces registered by all load sensors of the other insole;

Calibration factor $K_S$, like other calibration factors used for calculating person's weight at walking or running, can be determined in the process of system calibration. Yet, they can also be determined directly in the process of monitoring, when the person is known to carry no additional weight at a particular point of time, i.e., load sensors take up only person's known sole weight.

At a slow walking pace of up to 60 steps per minute, person's weight $P$, including additionally carried weight, can be calculated using formula:

$$P = K_W \cdot F\,,$$

where: $K_W$ is a calibration factor to be determined for a person with known weight at walking;

$F$ is the mean value of foot pressure force over one walking cycle, wherein:

$$F = (F_{1\max} + F_{2\max})/2\,,$$

where: $F_{1max}$ is the maximum value of summarized foot pressure forces registered by all load sensors of one

insole;

$F_{2max}$ is the maximum value of summarized foot pressure forces registered by all load sensors of the other insole,

whereat one cycle is assumed to consist of two consecutive steps made by one foot and the other.

At walking pace of 60 or more steps per minute, person's weight, including additionally carried weight, can be calculated by formula:

$$P = K_W \cdot F \cdot (1010 - 1.2 \cdot V - 0.026 \cdot V^2) \cdot 0.001,$$

where: $K_W$ is a calibration factor to be determined for a given person with known weight, who is walking at pace of up to 60 steps per minute;

$F$ is the mean value of foot pressure force over one walking cycle, wherein:

$$F = (F_{1\max} + F_{2\max})/2,$$

where: $F_{1max}$ is the maximum value of summarized foot pressure forces registered by all load sensors of one insole;

$F_{2max}$ is the maximum value of summarized foot pressure forces registered by all load sensors of the other insole;

$V$ is the number of steps per minute,

whereat one cycle is assumed to consist of two consecutive steps with one foot and the other;

The weight, including additionally carried weight, of a person at running can be calculated by formula:

$$P = K_R \cdot F \cdot (1090 - 4.4 \cdot V - 0.045 \cdot V^2) \cdot 0.001,$$

where: $K_R$ is a calibration factor, which is determined for a given person with known weight at running;

$F$ is the mean value of foot pressure force over one running cycle, wherein:

$$F = (F_{1\max} + F_{2\max})/2,$$

where: $F_{1max}$ is the maximum value of summarized foot pressure forces registered by all load sensors of one insole;

$F_{2max}$ is the maximum value of summarized foot pressure forces registered by all load sensors of the other insole;

$V$ is the number of steps per minute at a given running pace,

whereat one running cycle is assumed to consist of two consecutive steps made by one foot and the other.

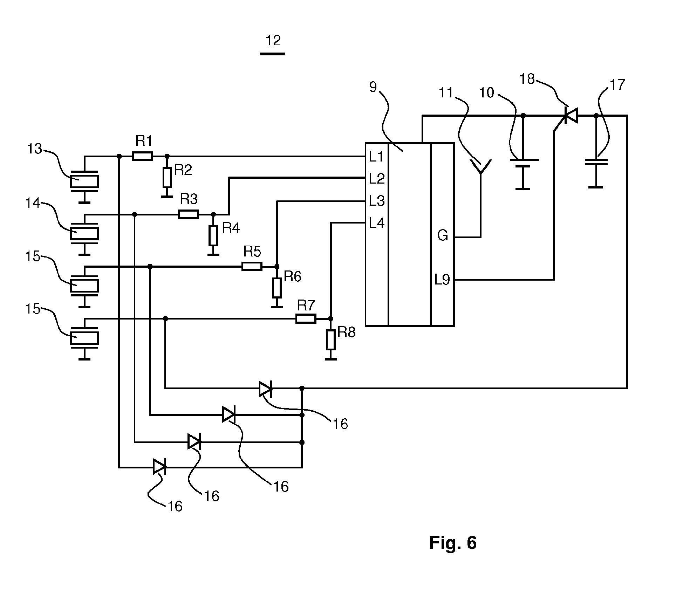

Another embodiment of device 12 for implementing the method using piezoelectric transducers as load sensors is shown in Fig. 6. Similarly to the preceding embodiment, right and left insoles 1 (see Fig. 1) comprise a first load sensor 2 located near the heel, a second load sensor 3 located near the toe, and two additional sensors 4 located essentially along trajectory 5 of support reaction force at walking. Load sensors 2, 3, and 4 in this embodiment are represented by piezoelectric transducers 13, 14, and 15, respectively, connected via matching resistors R1-R8 to input/output ports L1-L4 of microcontroller 9. Similarly to the embodiment shown in Fig.2, antenna output G of microcontroller 9 is connected to antenna 11.

The method for measuring person's weight in accordance with the present invention using piezoelectric transducers as load sensors is implemented as follows.

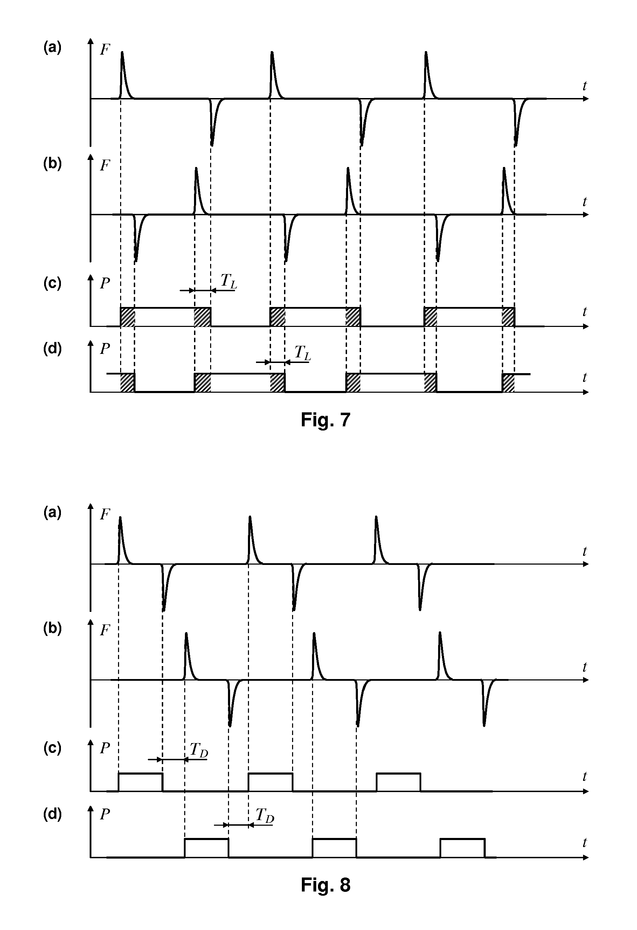

Similarly to the first implementation of the method, signals from load sensors 2, 3, and 4 of each insole 1, represented here by piezoelectric transducers 13, 14, and 15, respectively, are registered. Fig. 7 shows exemplary timing diagrams, which illustrate the temporal relationship of load sensor signals (e.g., piezoelectric transducers 13) from both insoles at walking, whereas Fig. 8 shows the same at running. Contrary to embodiment with strain transducers 7 (see Fig. 4 and Fig. 5), signals from the piezoelectric transducers, given their inherent amplitude-frequency response, have the form of distinctive spikes corresponding to the moment when the foot pressure is applied to piezoelectric transducer and removed from said transducer (a signal of reverse polarity). The availability of spikes with opposing polarities makes it possible to reconstruct the signal corresponding to the duration of support phase, and, similarly to the preceding example, to determine temporary relationships of signals from piezoelectric transducers of both insoles.

Thus, such type of motor activity as walking is characterized by signals shown in Fig. 7, where graph (a) corresponds to signal $F_1$ from piezoelectric transducer 13 (also known as sensor 2) of one insole 1, and graph (b) corresponds to signal $F_2$ from piezoelectric transducer 13 (also known as sensor 2) of the other insole 1. Graphs (c) and (d) in Fig. 7 show signals $P_1$ and $P_2$ corresponding to the duration of foot pressure on piezoelectric transducers 13 of one and the other insole 1, respectively. The rising edge of signals $P_1$ and $P_2$ in graphs (c) and (d) of Fig. 7 corresponds to signals $F_1$ and $F_2$ with positive polarity in graphs (a) and (b) of Fig. 7, respectively, while the falling edge of signals $P_1$ and $P_2$ in graphs (c) and (d) of Fig. 7 corresponds to signals $F_1$ and $F_2$ with negative polarity in graphs (a) and (b) of Fig. 7, respectively. Such type of motion activity as walking is characterized by alternating variation of signal values shown in graphs (c) and (d), and by the presence of their partial overlap (overlap time interval $T_L$).

Similarly, such type of motor activity as running is characterized by signals shown in Fig. 8, where graph (a) corresponds to signal $F_1$ from piezoelectric transducer 13 (also known as sensor 2) of one insole 1, and graph (b) corresponds to signal $F_2$ from piezoelectric transducer 13 (also known as sensor 2) of the other insole 1. Graphs (c) and (d) in Fig. 8 show signals $P_1$ and $P_2$ corresponding to the duration of foot pressure on piezoelectric transducers 13 of one and the other insole 1, respectively. The rising edge of signals $P_1$ and $P_2$ in graphs (c) and (d) of Fig. 8 corresponds to signals $F_1$ and $F_2$ with positive polarity in graphs (a) and (b) of Fig. 8, respectively, while the falling edge of signals $P_1$ and $P_2$ in graphs (c) and (d) of Fig. 8 corresponds to signals $F_1$ and $F_2$ with negative polarity in graphs (a) and (b) of Fig. 8, respectively. Such type of motion activity as walking is characterized by alternating variation of signal values shown in graphs (c) and (d) with no overlap in time. Conversely, a time gap designated as time interval $T_D$ between these signals is observed.

To measure person's weight, including additionally carried weight, mathematical relations from the first embodiment of the present invention can be used. In this case, maximum values of signals $F_1$ и $F_2$ from piezoelectric transducers are used as the pressure force exerted by foot on load sensors located in insoles. In case of standing, person's weight is determined by values of signals $F_1$ and $F_2$ generated by piezoelectric transducers at the beginning of standing activity and upon its termination. Apart from that, the implementation of the method is similar to the one described in the preceding example.

Devices 6 (Fig. 2) and 12 (Fig. 6), as well as portions thereof, can be mounted in insole 1 alongside with corresponding load sensors. The devices are energized by battery 10 which can be represented by a rechargeable battery. Additionally, the device can comprise a circuit for battery recharging performed in the process of walking or running, as shown in the example of device 12 (see Fig.6). Such a recharging circuit includes diodes 16, one for each piezoelectric transducer 13-15, storage capacitor 17, and thyristor 18 whose control input is coupled to input/output port L9 of microcontroller 9. One end of each diode 16 is connected to corresponding piezoelectric transducer 13-15, while all other ends are joined and connected to capacitor 17. In the process of recharging, microcontroller 9 switches off the registration mode of signals generated by foot pressure exerted on piezoelectric transducers, and sends said signals through diodes 16 to storage capacitor 17. In this mode, microcontroller 9 opens thyristor 18, thereby connecting charged capacitor 17 to battery 10. So, when a person is walking or running in shoes with device 12 and piezoelectric transducers 13-15 mounted in shoe insoles, battery 10 is recharged.

The method in accordance with the present invention enables a real-time measurement of human weight, with type of motor activity and additionally carried weight taken into account.

Furthermore, the data registered by load sensors mounted in insoles, as described above, may also be used to detect the defects of person's musculoskeletal system, flat footedness, as well as to determine the slope of travel surface and its hardness, degree of comfort of the footwear worn, identification of gait specific for a person, etc. The method also enables a continuous monitoring of traveling pace, motion speed and acceleration, covered distance and duration of travel.

## Claims

1. A method for determining weight of a human, the method comprising:

   registering signals from load sensors disposed in shoe insoles, wherein each insole comprises two load sensors, a first load sensor being disposed in a heel region and a second load sensor being disposed in a toe region;
   determining a type of motor activity by using the signals from the load sensors and by determining correspondence in time between the signals; and
   determining the weight of the human by summing values of the signals from the load sensors and based on the type of the motor activity, wherein determining the weight comprises determining additionally carried weight.

2. The method of Claim 1, wherein the type of the motor activity is determined as "walking" if the signals from the load sensors in both insoles exhibit periodic variations of the values of the signals and the signals from the load sensors of different insoles partially overlap in time.

3. The method of Claim 1, wherein the type of the motor activity is determined as "running" if the signals from the load sensors in both insoles exhibit periodic variations of the values of the signals and the signals from the load sensors of different insoles do not overlap in time.

4. The method of Claim 1, wherein the type of the motor activity is determined as "standing" if the signals from the load sensors in both insoles exceed a predetermined value and the signals from different insoles overlap in time.

5. The method of Claim 1, wherein the weight of the person, and the additionally carried weight, walking at a pace of up to 60 steps per minute is determined as:

$$P = K_W \cdot F \,,$$

   wherein: $K_W$ is a calibration factor determined for a person with a known weight at walking;
   $F$ is a mean value of a foot pressure force over one walking cycle, wherein:

$$F = (F_{1\max} + F_{2\max})/2 \,,$$

   where: $F_{1max}$ is a maximum value of summarized foot pressure forces registered by all the load sensors in one insole; and
   $F_{2max}$ is a maximum value of summarized foot pressure forces registered by all the load sensors of the other insole;
   wherein one walking cycle consists of two consecutive steps made by one foot and then another.

6. The method of Claim 1, wherein the weight of the person, and the additionally carried weight, walking at a pace of 60 or more steps per minute is determined as:

$$P = K_W \cdot F \cdot (1010 - 1.2 \cdot V - 0.026 \cdot V^2) \cdot 0.001 \,,$$

   wherein: $K_W$ is a calibration factor for a given person with a known weight walking at a pace of up to 60 steps per minute;
   $F$ is a mean value of foot pressure force over one walking cycle, wherein:

$$F = (F_{1\max} + F_{2\max})/2 \,,$$

   where: $F_{1max}$ is a maximum value of summarized foot pressure forces registered by all the load sensors of one insole;
   $F_{2max}$ is the maximum value of summarized foot pressure forces registered by all the load sensors of the other insole; and

$V$ is a number of steps per minute;
wherein one walking cycle consists of two consecutive steps made by one foot and then another.

7. The method of Claim 1, wherein the weight of the person, and the additionally carried weight, during running is determined as:

$$P = K_R \cdot F \cdot (1090 - 4.4 \cdot V - 0.045 \cdot V^2) \cdot 0.001 ,$$

wherein: $K_R$ is a calibration factor determined for a given person with a known weight at running;
$F$ is a mean value of a foot pressure force over one running cycle, wherein:

$$F = (F_{1\max} + F_{2\max})/2 ,$$

wherein: $F_{1max}$ is a maximum value of summarized foot pressure forces registered by all the load sensors of one insole;
$F_{2max}$ is maximum value of summarized foot pressure forces registered by all the load sensors of the other insole; and
$V$ a number of steps per minute;
wherein one running cycle consists of two consecutive steps made by one foot and then another.

8. The method of Claim 1, wherein the weight of the person, and the additionally carried weight, while standing is determined as:

$$P = K_S \cdot F ,$$

wherein: $K_S$ is a calibration factor determined for a given person with a known weight at standing;
$F$ is a mean value of a foot pressure force at standing, wherein:

$$F = (F_{1\max} + F_{2\max})/2 ,$$

wherein: $F_{1max}$ is a maximum value of summarized foot pressure forces registered by all the load sensors of one insole; and
$F_{2max}$ is the maximum value of summarized foot pressure forces registered by all the load sensors of the other insole.

9. The method of Claim 1, further comprising providing additional load sensors in each insole, the additional sensors being disposed along a trajectory of a support reaction force at walking between the first load sensor and the second load sensor, detecting signals from the additional load sensors and using such signals in addition to using the signals from the first and the second load sensors.

10. An insole for determining the weight of the human according toClaim 1, comprising a first load sensor disposed in the insole in a heel region, and a second load sensor disposed in the insole in a toe region, the first and the second load sensors being capable of generating signals detecting a pressure force applied by the person’s foot.

11. The insole of Claim 10 further comprising an analog-to-digital converter and a transceiver to convert the signals from the load sensors to a digital form and transmit them to an external processing unit.

3
1
1
3
4
4
5
5
4
4
2
2

Fig. 1

6
8
8
9
11
10
7
8
8
7
7
7
L1
L2
L3
L4
L5
L6
L7
L8
G

Fig. 2

(a)
$F_1$
$t$

(b)
$F_2$
$t$

**Fig. 3**

(a)
$F_1$
$t$
$T_L$
$T_L$
$T_L$
$T_L$
$T_L$
(b)
$F_2$
$t$

**Fig. 4**

(a)
$F_1$
$t$
$T_D$
$T_D$
$T_D$
$T_D$
(b)
$F_2$
$t$

**Fig. 5**

12
9
11
10
18
17
13
14
15
15
R1
R2
R3
R4
R5
R6
R7
R8
L1
L2
L3
L4
G
L9
16
16
16
16
16

Fig. 6

(a)
F
t
(b)
F
t
(c)
P
$T_L$
t
(d)
P
$T_L$
t

Fig. 7

(a)
F
t
(b)
F
t
(c)
P
$T_D$
t
(d)
P
$T_D$
t

Fig. 8

## INTERNATIONAL SEARCH REPORT

International application No.
PCT/RU 2014/000138

A. CLASSIFICATION OF SUBJECT MATTER

***G01G 3/13*** *(2006.01)* ***A43B 17/00*** *(2006.01)*
***A61B 5/22*** *(2006.01)* ***G06F 19/00*** *(2011.01)*

According to International Patent Classification (IPC) or to both national classification and IPC

B. FIELDS SEARCHED

Minimum documentation searched (classification system followed by classification symbols)

A61B 5/103, A43B 17/00, G06F 19/00, G01G 3/13, A61B 5/22

Documentation searched other than minimum documentation to the extent that such documents are included in the fields searched

Electronic data base consulted during the international search (name of data base and, where practicable, search terms used)

Patentscope, USPTO DB, Espacenet, DWPI, CIPO (Canada PO), SIPO DB, AIPN, DEPATISnet, NCBI (PubMed), VINITI.RU, SCSML.FSSI.RU

C. DOCUMENTS CONSIDERED TO BE RELEVANT

| Category* | Citation of document, with indication, where appropriate, of the relevant passages | Relevant to claim No. |
|---|---|---|
| A | US 2012/0253234 A1 (MING YOUNG BIOMEDICAL CORP.) 04.10.2012 | 1-11 |
| A | RU 103064 U1 (STEPANOV OLEKSANDR OLEKSANDROVICH) 27.03.2011 | 1-11 |
| A | SAZONOVA Nadezhda A. et al. Predection of Bodyweight and Energy Expenditure Using Point Pressure and Foot Acceleration Measurements. The Open Biomedical Engineering Journal, 2011, 5, pp. 110-115 | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 August 2014 (20.08.2014) | 21 August 2014 (21.08.2014) |
| Name and mailing address of the ISA/ RU | Authorized officer |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002090216 B
- WO 200136051 A
- US 6273863 B
- WO 2009059134 A